(19) Europäisches Patentamt
European Patent Office
Office européen des brevets

(11) Publication number: **0 393 641 A2**

# (12) EUROPEAN PATENT APPLICATION

(21) Application number: **90107375.9**

(22) Date of filing: **18.04.90**

(51) Int. Cl.[5]: **C07D 237/16, C07D 237/18, A01N 43/58**

(30) Priority: **19.04.89 JP 101437/89**

(43) Date of publication of application: **24.10.90 Bulletin 90/43**

(84) Designated Contracting States: **DE FR GB IT**

(71) Applicant: **OTSUKA KAGAKU KABUSHIKI KAISHA**
**2-27, Ote-dori 3-chome Chuo-ku**
**Osaka-shi Osaka-fu 540(JP)**

(72) Inventor: **Takao, Hisashi**
**135-11, Aza-Hara, Tainohama**
**Kitajima-cho, Itano-gun, Tokushima-ken(JP)**

(74) Representative: **Barz, Peter, Dr. et al**
**Patentanwälte Dipl.-Ing. G. Dannenberg Dr. P. Weinhold, Dr. D. Gudel Dipl.-Ing. S. Schubert, Dr. P. Barz Siegfriedstrasse 8**
**D-8000 München 40(DE)**

## (54) Pyridazinone derivatives.

(57) The present invention provides a novel pyridazinone derivative represented by the formula

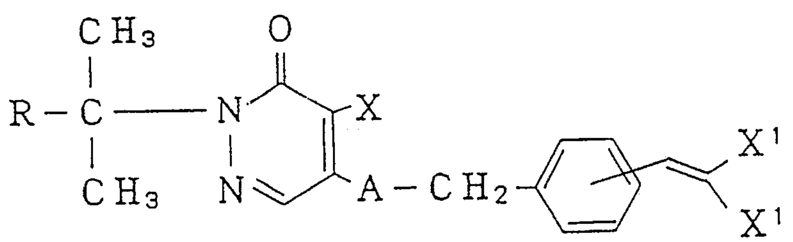

(I)

wherein R is a hydrogen atom or methyl, X is a halogen atom, A is an oxygen atom or a sulfur atom, and $X^1$ is a halogen atom; a insecticidal, miticidal and fungicidal composition for agricultural and horticultural use comprising an effective amount of said pyridazinone derivative; and method for killing harmful insects, mites and bacteria comprising applying to a plant an effective amount of said pyridazinone derivative.

EP 0 393 641 A2

## PYRIDAZINONE DERIVATIVES

The present invention relates to novel pyridazinone derivatives and insecticidal, miticidal and fungicidal compositions containing said derivative as an active principle.

Various pyridazinone derivatives comprising benzyl ether group or benzylthio ether group have been reported. Particularly, the pyridazinone derivatives disclosed in Japanese Unexamined Patent Application Nos. 152870/1983, 39874/1984, 70673/1984, 98064/1984, 4173/1985, 54319/1985, 148106/1986, 17570/1986 and 229802/1986 prove to display insecticidal, miticidal and nematocidal effect as well as fungicidal effect. However, these derivatives, when used at practical concentrations, do not possess sufficiently high ability to kill a variety of major harmful insects and other harmful insects which are difficult to get rid of due to development of their resistance. Therefore, they are inadequate when used as exterminators for harmful insects in agricultural and horticultural use.

It is, therefore, an object of the present invention to provide an insecticidal, miticidal and fungicidal compound which is active on a variety of major harmful insects as well as other harmful insects which are difficult to get rid of due to development of their resistance.

It is another object of the present invention to provide an insecticidal, miticidal and fungicidal composition containing the above compound.

The present inventors made efforts to develop compounds useful as preventive for diseases and harmful insects in agricultural and horticultural use, and found that the pyridazinone derivatives of the formula (I) have not only fungicidal activity but also excellent insecticidal and miticidal activity and show broad insecticidal spectrum. That is, the above pyridazinone derivatives have excellent ability to kill insects of the order Hemiptera including aphids and insects of the order mites and also have extremely potent insecticidal activity on insects of the order Lepidoptera including cabbage armyworms and common cabbageworms. Further said derivatives are significantly effective against diamond-back moths, thrips, etc. which are regarded as insects difficult to control due to their developed resistance. From this viewpoint, said derivatives can be one of the most effective exterminators for diseases and harmful insects in agricultural and horticultural use. The present invention was thus accomplished.

The present invention provides novel pyridazinone derivatives which have not been reported in publications and are represented by the following formula (I).

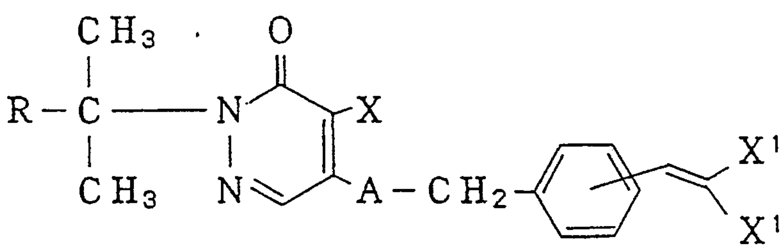

(I)

wherein R is a hydrogen atom or methyl, X is a halogen atom, A is an oxygen atom or a sulfur atom, and $X^1$ is a halogen atom.

Examples of halogen atoms represented by X and $X^1$ are chlorine, bromine, etc.

The pyridazinone derivatives of the formula (I) can be produced by various methods. Typically, the method represented by the following Reaction scheme 1 may be employed.

<Reaction scheme 1>

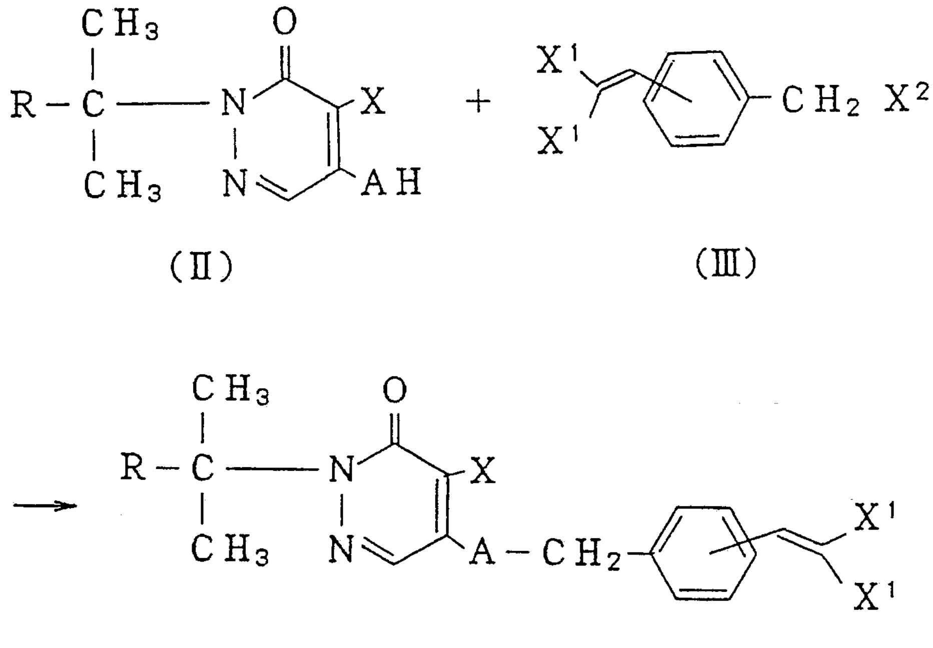

(II) (III)

(I)

In the foregoing formulas, R, X, A and $X^1$ are as defined above, and $X^2$ is a halogen atom such as chlorine, bromine or fluorine atom.

The present compound is prepared by reacting a pyridazinone derivative of the formula (II) with a benzyl halide of the formula (III) in the presence of an acid acceptor.

The above reaction is conducted in a solvent. Examples of such solvent include alcohols such as methanol, ethaol, etc., ethers such as ethyl ether, butyl ether, tetrahydrofuran, dioxane, etc., nitriles such as acetonitrile, propionitrile, etc., ketones such as acetone, methyl ethyl ketone, etc., halogenated hydrocarbons such as dichloromethane, dichloroethane, etc., amides such as dimethylformamide, dimethylacetamide, etc., and so on.

While the ratio of said benzyl halide of the formula (III) to said pyridazinone derivative of the formula (II) is not specifically limited but may be selected from a broad range, and it is generally preferable to use about 0.5 to about 2 moles, preferably about 1 to about 1.5 moles, of the benzyl halide (III) per mole of the pyridazinone derivative (II). The acid acceptor may be selected from a wide range of known compounds including tertiary amines such as triethylamine, pyridine, etc., alkali metal carbonates such as sodium carbonate, potassium carbonate, etc., alkali metal hydroxides such as sodium hydroxide, potassium hydroxide, etc., and alkali metal hydrides such as sodium hydride, potassium hydride and so on. The amount of said acid acceptor is generally about 1 to about 2 moles and preferably about 1 to about 1.2 moles per mole of the pyridazinone derivative (II). This reaction generally proceeds preferably at about 10 to about 50 °C and generally goes to completion in about 1 to about 5 hours.

The compound (II) used as a starting material in Reaction scheme 1 is a known compound and can be easily produced by a known method, for example by a method described in Angew. Chem. Internat. Edit., 4(4), 292-300 (1965).

The compound (III) used as the other starting material can be easily prepared according to the following Reaction scheme 2.

<Reaction scheme 2>

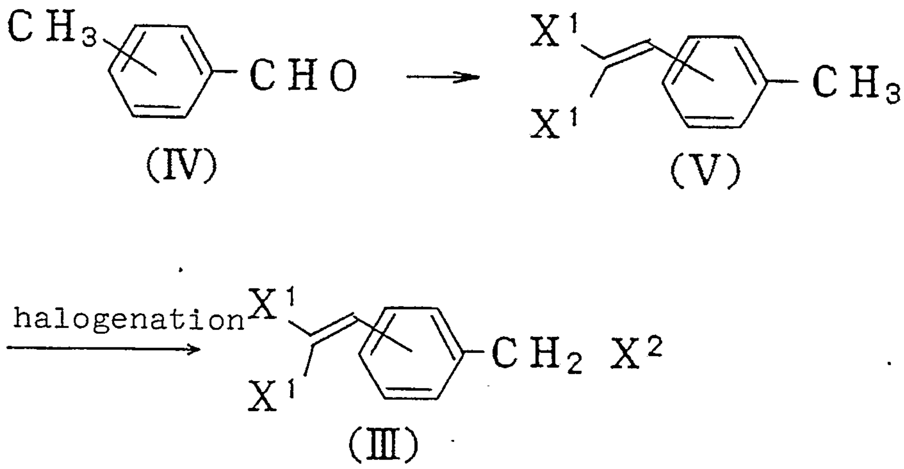


In the above formulas, $X^1$ and $X^2$ are as defined above.

In Reaction scheme 2, the compound (V) can be prepared from tolualdehyde (IV) by a known procedure. For example, an intermediate reaction product of carbon tetrahalogenide and triphenyl phosphine is reacted with tolualdehyde (IV) to give the compound (V). The details of this process are described in J.A.C.S., 84, 1745-1747 (1962). This reaction will be illustrated in REFERENCE EXAMPLE 1 to be described later.

The compound (III) can be prepared in high yield by halogenating a compound (V) in a conventional manner, for example, using chrorine, bromine, N-halogenated phthalimide or the like, as will be described below in REFERENCE EXAMPLE 2.

The compound of the present invention as produced by the above method can be easily isolated and purified from the reaction mixture by conventional separating methods such as solvent extraction solvent dilution, distillation, recrystallization, column chromatography and so on. In accordance with the above-described production method, and the compound of the present invention can be produced in high yield and high purity.

Typical examples of the compounds of the present invention thus obtaind are as follows:

2-t-Butyl-4-chloro-5-[4-(β,β-dichlorovinyl)]benzylthio-3(2H)-pyridazinone (Compound 1)
2-t-Butyl-4-chloro-5-[4-(β,β-dichlorovinyl)]benzyloxy-3(2H)-pyridazinone (Compound 2)
2-t-Butyl-4-chloro-5-[4-(β,β-dibromovinyl)]benzylthio-3(2H)-pyridazinone (Compound 3)
2-t-Butyl-4-chloro-5-[3-(β,β-dichlorovinyl)]benzylthio-3(2H)-pyridazinone (Compound 4)
2-t-Butyl-4-chloro-5-[2-(β,β-dichlorovinyl)benzylthio-3(2H)-pyridazinone (Compound 5)
2-t-Butyl-4-chloro-5-[3-(β,β-dichlorovinyl)]benzyloxy-3(2H)-pyridazinone (Compound 6)
2-t-Butyl-4-chloro-5-[2-(β,β-dichlorovinyl)]benzyloxy-3(2H)-pyridazinone (Compound 7)
2-t-Butyl-4-bromo-5-[4-(β,β-dichlorovinyl)]benzylthio-3(2H)-pyridazinone (Compound 8)
2-t-Butyl-4-bromo-5-[4-(β,β-dibromovinyl)]benzylthio-3(2H)-pyridazinone (Compound 9)
2-t-Butyl-4-bromo-5-[2-(β,β-dichlorovinyl)]benzylthio-3(2H)-pyridazinone (Compound 10)
2-t-Butyl-4-bromo-5-[4-(β,β-dichlorovinyl)]benzyloxy-3(2H)-pyridazinone (Compound 11)
2-t-Butyl-4-bromo-5-[2-(β,β-dichlorovinyl)]benzyloxy-3(2H)-pyridazinone (Compound 12)
2-Isopropyl-4-chloro-5-[4-(β,β-dichlorovinyl)]benzylthio-3(2H)-pyridazinone (Compound 13)
2-Isopropyl-4-chloro-5-[4-(β,β-dichlorovinyl)]benzyloxy-3(2H)-pyridazinone (Compound 14)
2-Isopropyl-4-chloro-5-[2-(β,β-dichlorovinyl)]benzylthio-3(2H)-pyridazinone (Compound 15)
2-Isopropyl-4-chloro-5-[3-(β,β-dichlorovinyl)]benzylthio-3(2H)-pyridazinone (Compound 16)
2-Isopropyl-4-chloro-5-[3-(β,β-dichlorovinyl)]benzyloxy-3(2H)-pyridazinone (Compound 17)
2-Isopropyl-4-bromo-5-[2-(β,β dichlorovinyl)]benzylthio-3(2H)-pyridazinone (Compound 18)
2-Isopropyl-4-chloro-5-[4-(β,β-dibromovinyl)]benzylthio-3(2H)-pyridazinone (Compound 19)
2-Isopropyl-4-bromo-5-[4-(β,β-dichlorovinyl)]benzylthio-3(2H)-pyridazinone (Compound 20)

The compound according to the present invention has high insecticidal and miticidal activity as well as excellent fungicidal activity and effectively acts on resistant harmful insects, so that it can be advantageously used for controlling diseases and harmful insects in crops such as vegetables, orchard trees, etc.

Thus, the present invention further provides an insecticidal, miticidal and fungicidal composition comprising the compound (I) as an active ingredient.

For use as an insecticidal, miticidal and/or fungicidal composition, the compound (I) according to the present invention can be made available in various application forms such as an emulsion, aqueous

solution, suspension, fine powder, powder, wettable powder, paste, foamable spray composition, microencapsulated preparation, aerosol preparation, natural or synthetic matrix-impregnated preparation, fumigant composition, ultra-low volume concentrate, and so on. In the preparation of such application forms, various surfactants which are conventionally used in the art can be used for emulsifying, dispersing, suspending, and/or foaming purposes. Such surfactants include nonionic surfactants such as polyoxyethylene alkyl ethers, polyoxyethylene alkyl esters, polyoxyethylene sorbitan alkyl esters, sorbitan alkyl esters, etc., anionic surfactants such as alkyl benzenesulfonates, alkyl sulfosuccinates, alkyl sulfates, polyoxyethylene alkyl sulfates, aryl sulfonates, lignin sulfite, and so on. As a vehicle, diluent or carrier, various organic solvents, aerosol propellants, naturally-occurring mineral and vegetable materials, and synthetic compounds which are conventional in this filed of art can be utilized. As preferred examples of said organic solvent, there may be mentioned benzene, toluene, xylene, ethylbenzene, chlorobenzene, alkylnaphthalenes, dichloromethane, chloroethylene, cyclohexane, cyclohexanone, acetone, methyl ethyl ketone, methyl isobutyl ketone, alcohols, dimethylformamide, dimethyl sulfoxide, acetonitrile, mineral oil fractions, and so on. The aerosol propellants include, among others, propane, butane, halogenated hydrocarbons, nitrogen, carbon dioxide and so on. The mineral materials include kaolin, talc, bentonite, diatomaceus earth, clay, monmorillonite, chalk, calcite, pumice, sepiolite, dolomite, and so on. The vegetable materials include walnut shells, tobacco stalks, sawdust and so on. Examples of said synthetic compounds include alumina, silicate, polysaccharides and so on. As adhesive agents, carboxymethylcellulose, gum arabic, polyvinyl alcohol, polyvinyl acetate, and so on may be employed. Furthermore, said various compositions may be tinted with an organic or inorganic colorant. In the various compositions provided by the present invention, the compound of the present invention may be incorporated in a proportion of about 0.1 to about 95 percent by weight and preferably about 0.5 to about 90 percent by weight.

Such composition may be used as such or after dilution with a suitable carrier or a vehicle such as water to a concentration suited for the intended application. Thus, the dilution may contain about 0.00001 to about 100 percent by weight and, preferably, about 0.0001 to about 10 percent by weight of the compound of the present invention. The application amount varies with the severity of infestation with mites, etc., weather, and other conditions and cannot be generally specified. However, the composition of the present invention is generally used in an amount of about 0.1 to about 10 kg, preferably about 0.1 to about 1 kg, per hectare, calculated as the compound of the formula (I).

The following reference examples, examples and test examples are intended to illustrate the present invention in further detail.

## REFERENCE EXAMPLE 1

To 200 ml of anhydrous carbon tetrachloride were added 12.0 g (0.1 mole) of p-tolualdehyde and 52.45 g (0.2 mole) of triphenyl phosphine, and the mixture was stirred at 60° C for 2 hours. Then the carbon tetrachloride was removed from the reaction mixture, followed by addition of 300 ml of n-hexane. The triphenyl phosphine oxide precipitated was filtered off under reduced pressure. The filtrate was concentrated, and the residue was distilled under reduced pressure to give 10.85 g of 4-($\beta$,$\beta$-dichlorovinyl)-toluene as a pale yellow oil (yield: 85%).

B.P.: 70-71° C/2 mmHg

NMR ($CDCl_3$); δppm:

2.30(s, 3H, $-CH_3$), 6.70(s, 1H, $CH=CCl_2$), 6.90-7.40(m, 4H, aromatic H)

## REFERENCE EXAMPLE 2

To 700 ml of carbon tetrachloride were added 210 g (1.11 mole) of 4-($\beta$,$\beta$-dichlorovinyl)toluene prepared in REFERENCE EXAMPLE 1 and 213.6 g (1.2 mole ) of N-bromophthalimide, and the mixture was refluxed with stirring for 3 hours. The reaction mixture was cooled to room temperature, and solid phthalimide was filtered off under reduced pressure. Then the filtrate was washed with 5% aqueous sodium hydrocarbonate and dried over anhydrous magnesium sulfate. The solvent was evaporated and the residue was distilled under reduced pressure to give 259.6 g of 4-($\beta$,$\beta$-dichlorovinyl)phenylbenzyl bromide as a yellow oil (yield: 88%).

B.P.: 114-116° C/2 mmHg

NMR ($CDCl_3$); δppm:
4.38(s, 2H, $CH_2Br$), 6.70(s, 1H, $CH=CCl_2$), 7.10-7.52(m, 4H, aromatic H)

EXAMPLE 1

To 15 ml of ethanol were added 2.195 g (0.01 mole) of 2-t-butyl-4-chloro-5-mercapto-3(2H)-pyridazinone and 1.06 g (0.01 mole) of anhydrous sodium carbonate with stirring at room temperature, and 2.66 g (0.01 mole) of 4-(β,β-dichlorovinyl)benzyl bromide was added dropwise thereto. After the addition was completed, the mixture was stirred at room temperature for 4 hours. A 100 ml quantity of benzene and 50 ml of water were then added to the reaction mixture, and the organic layer was washed with 5% aqueous sodium hydroxide and saturated brine, followed by drying over anhydrous magnesium sulfate. The solvent was then evaporated to give a crude product. The crude product was recrystallized from n-hexane/benzene, giving 3.65 g of 2-t-butyl-4-chloro-5-[4-(β,β-dichlorovinyl)] benzylthio-3(2H)-pyridazinone as white crystal.
M.P.: 106-107.8° C
NMR ($CDCl_3$); δppm:
1.58(s,9H, alkyl), 4.16(s, 2H, $CH_2S$), 6.70(s, 1H, CH=C), 7.16-7.48(m, 4H, Ar-H), 7.42(s, 1H, CH=N)
Based on the above physical and analytical data, the compound was identified as the compound (Compound 1) of the following formula:

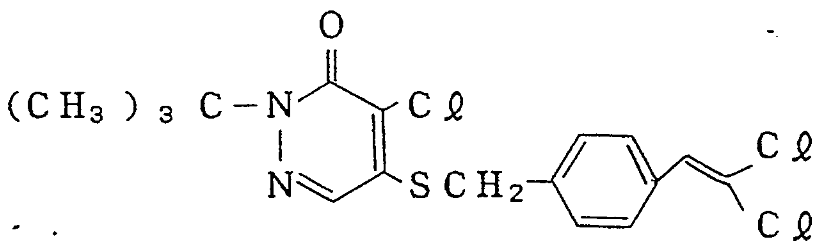

EXAMPLE 2

To 15 ml of acetonitrile were added 2.05 g (0.01 mole) of 2-t-butyl-4-chloro-5-hydroxy-3(2H)-pyridazinone and 1.38 g (0.01 mole) of anhydrous potassium carbonate, and the mixture was stirred at 40° C for 1 hour. A 2.66 g (0.01 mole) quantity of 4-(β,β-dichlorovinyl)benzylbromide was added dropwise to the mixture, followed by refluxing with stirring for 5 hours. Then the acetonitrile was removed, and the residue was diluted with benzene. The organic layer was washed with 5% aqueous sodium hydroxide and saturated brine and then dried over anhydrous magnesium sulfate. After the solvent was evaporated, the residue was recrystallized from n-hexane/benzene, giving 3.12 g of 2-t-butyl-4-chloro-5-[4-(β,β-dichlorovinyl)]benzyloxy-3(2H)-pyridazinone as white crystal.
M.P.: 153.2-154° C
NMR ($CDCl_3$); δppm:
1.58(s, 9H, alkyl), 5.68(s, 2H, $CH_2O$), 6.66(s, 1H, CH=C), 7.08-7.48(m, 4H, Ar-H), 7.50(s, 1H, CH=N)
Based on the above physical and analytical data, the compound was identified as the compoud (Compound 2) of the following formula:

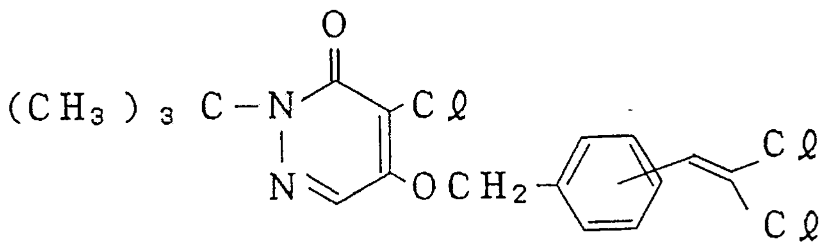

EXAMPLES 3 to 20

The compounds shown in Table 1 were prepared in the same manner as in Example 1 or 2.

TABLE 1

| Compound No | Chemical Structure | Appearance |
| --- | --- | --- |
| 3 | 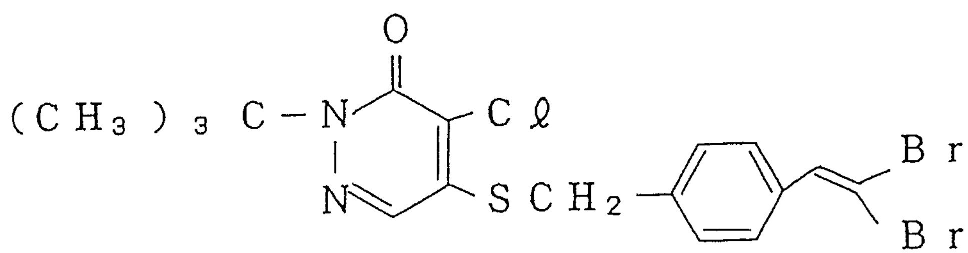 | White Cryatal M.P. : 120～121. 5℃ |
| 4 | 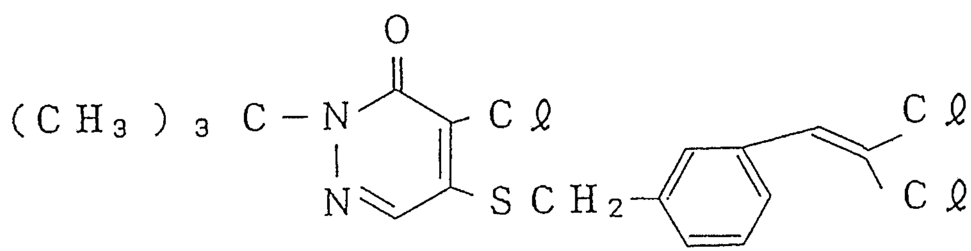 | Yellow Oil |
| 5 | 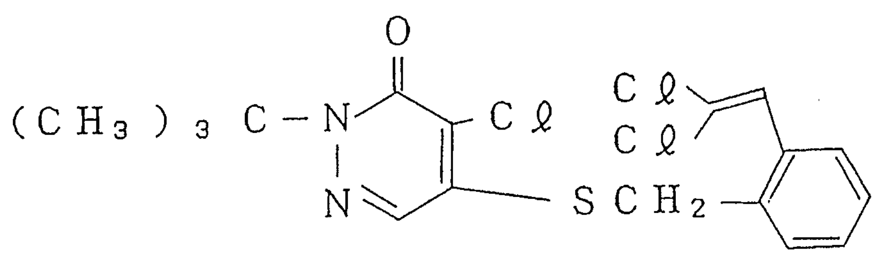 | White Crystal M.P.: 115～116℃ |

| Compound No. | Chemical Structure | Appearance |
|---|---|---|
| 6 | 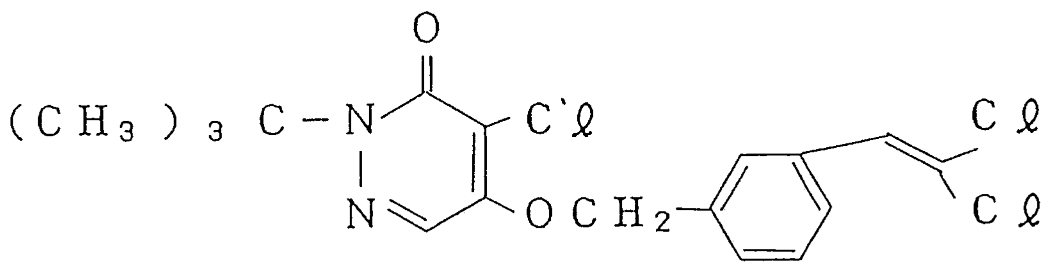 | White Crystal M.P.: 136~137℃ |
| 7 | 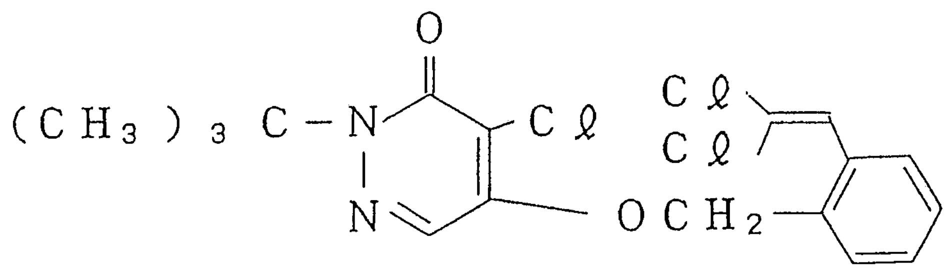 | Yellow Oil |
| 8 | 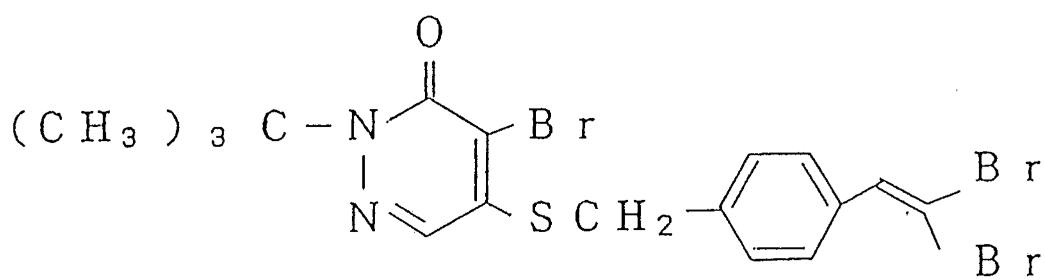 | Yellow Oil |

| Compound No. | Chemical Structure | Appearance |
| --- | --- | --- |
| 9 | 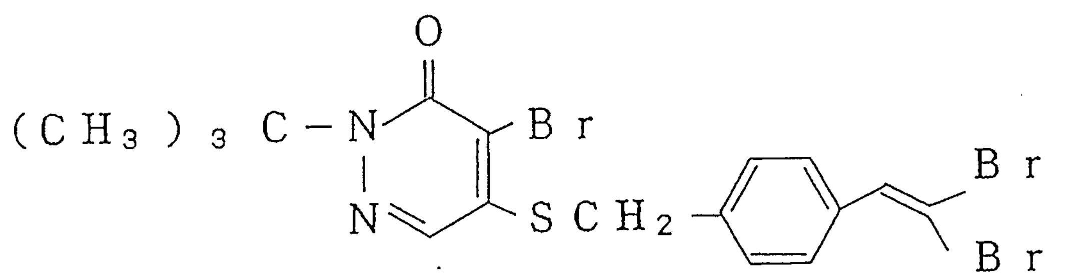 | Yellow Oil |
| 10 | 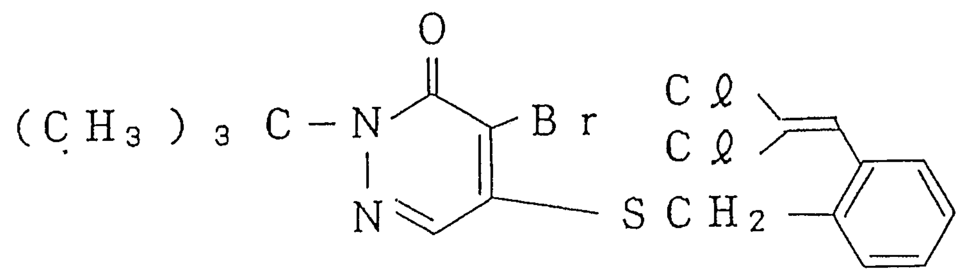 | Yellow Oil |
| 11 | 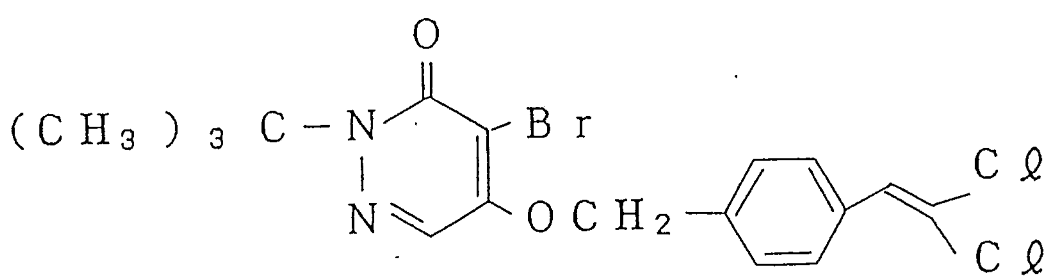 | White Crystal M.P.: 160~161.3℃ |

| Compound No. | Chemical Structure | Appearance |
|---|---|---|
| 12 | 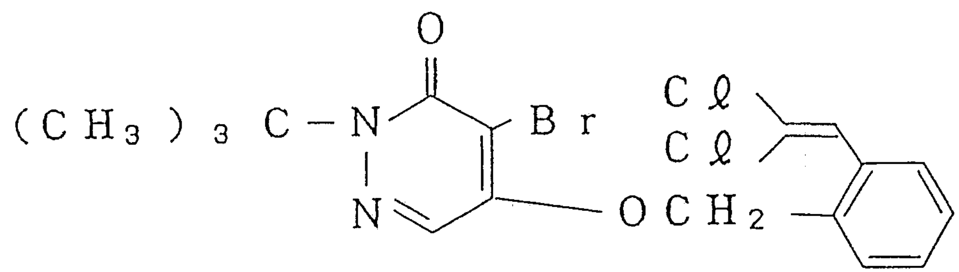 | White Crystal M.P.: 155~157.8℃ |
| 13 | 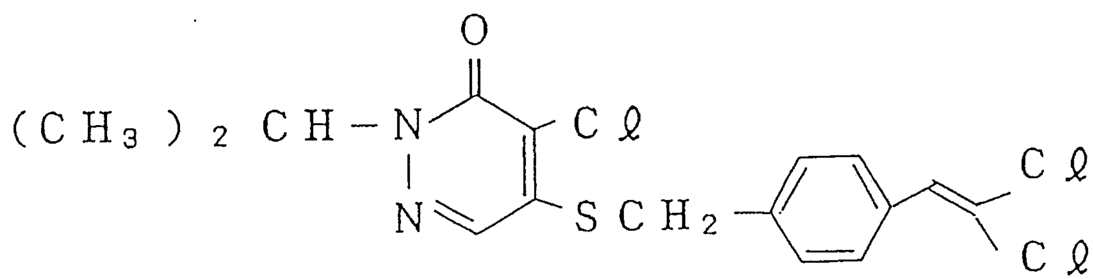 | White Crystal M.P.: 136~137.3℃ |
| 14 | 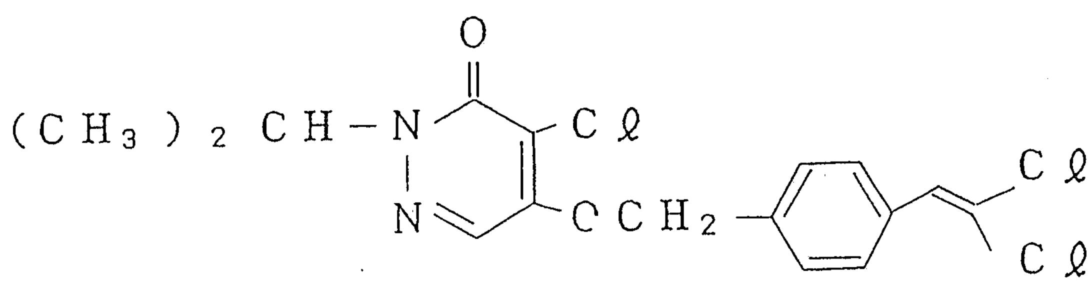 | White Crystal M.P.: 167~168.3℃ |

| Compound No. | Chemical Structure | Appearance |
|---|---|---|
| 15 | 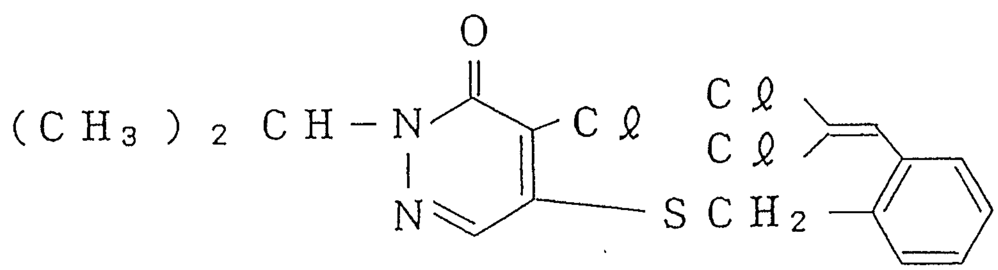 | White Crystal<br>M.P.: 140～141℃ |
| 16 | 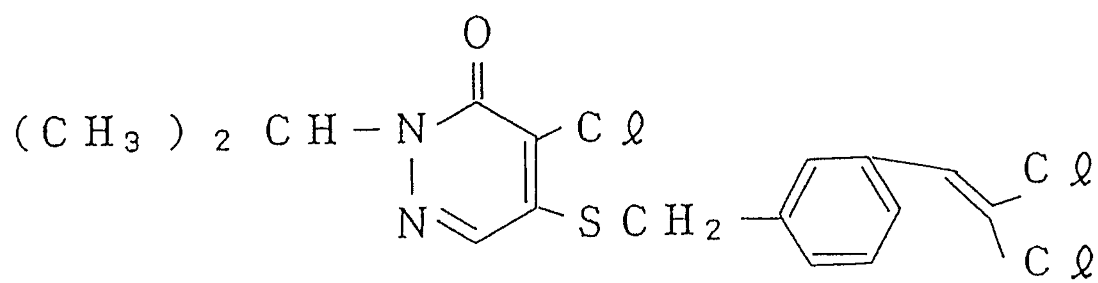 | Yellow Oil |
| 17 | 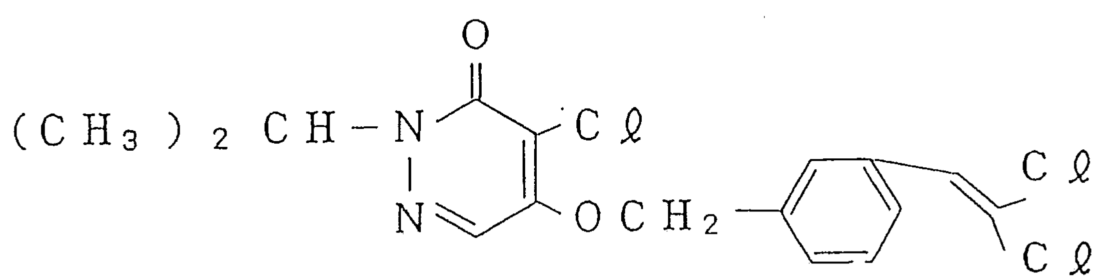 | White Crystal<br>M.P.: 139～140℃ |

| Compound No. | Chemical Structure | Appearance |
|---|---|---|
| 18 | 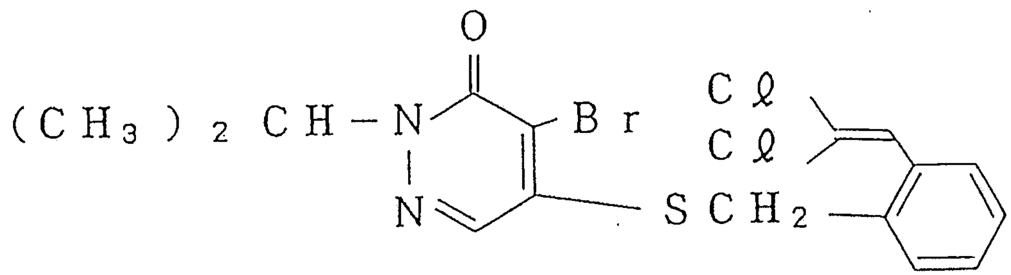 | Yellow Oil |
| 19 | 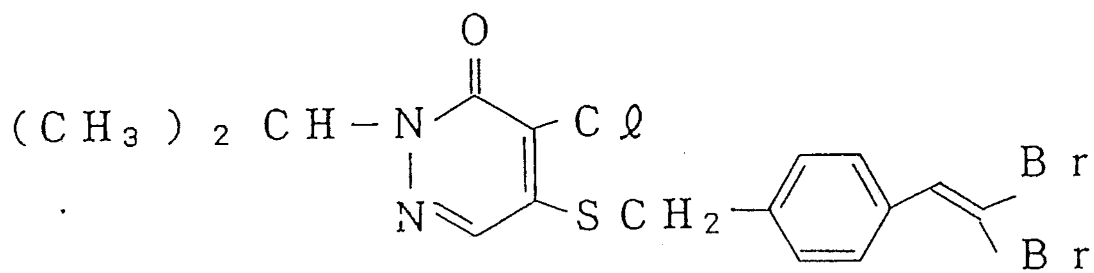 | White Crystal M.P.: 161.5~163℃ |
| 20 | 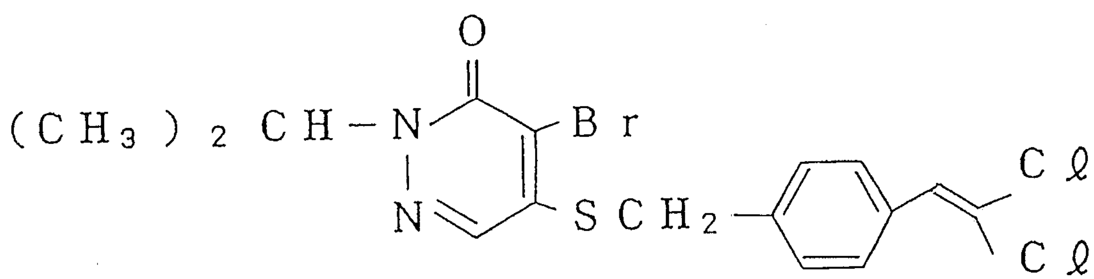 | Yellow Oil |

| Compound No. | NMR ($CDCl_3$) Data, δ ppm |
|---|---|
| 3 | 1.58 (s、9H、alkyl )<br>4.18 (s、2H、$CH_2$ S)<br>7.38 (s、1H、CH=C)<br>7.20~7.90 (m、4H、aromatic H )<br>7.48 (s、1H、CH=N) |
| 4 | 1.58 (s、9H、alkyl )<br>4.20 (s、2H、$CH_2$ S)<br>6.68 (s、1H、CH=C)<br>7.10~7.50 (m、4H、aromatic H )<br>7.46 (s、1H、CH=N) |
| 5 | 1.58 (s、9H、alkyl )<br>4.18 (s、2H、$CH_2$ S)<br>6.70 (s、1H、CH=C)<br>7.10~7.50 (m、4H、aromatic H )<br>7.48 (s、1H、CH=N) |
| 6 | 1.58 (s、9H、alkyl )<br>5.18 (s、2H、$CH_2$ O)<br>6.70 (s、1H、CH=C)<br>7.10~7.50 (m、4H、aromatic H )<br>7.66 (s、1H、CH=N) |

| Compound No. | NMR ($CDCl_3$) Data , δ ppm |
|---|---|
| 7 | 1.58 (s、9H、alkyl )<br>5.16 (s、2H、$CH_2$ O)<br>6.90 (s、1H、CH=C)<br>7.10~7.48 (m、4H、aromatic H )<br>7.62 (s、1H、CH=N) |
| 8 | 1.58 (s、9H、alkyl )<br>4.18 (s、2H、$CH_2$ S)<br>6.70 (s、1H、CH=C)<br>7.14~7.50 (m、4H、aromatic H )<br>7.38 (s、1H、CH=N) |
| 9 | 1.58 (s、9H、alkyl )<br>4.20 (s、2H、$CH_2$ S)<br>7.38 (s、1H、CH=C)<br>7.16~7.60 (m、4H、aromatic H )<br>7.48 (s、1H、CH=N) |
| 10 | 1.58 (s、9H、alkyl )<br>4.18 (s、2H、$CH_2$ S)<br>6.70 (s、1H、CH=C)<br>7.10~7.50 (m、4H、aromatic H )<br>7.38 (s、1H、CH=N) |

| Compound No. | NMR ($CDCl_3$) Data , δ ppm |
|---|---|
| 11 | 1.58 (s、9H、alkyl )<br>5.20 (s、2H、$CH_2O$)<br>6.70 (s、1H、CH=C)<br>7.10~7.50 (m、4H、aromatic H )<br>7.40 (s、1H、CH=N) |
| 12 | 1.58 (s、9H、alkyl )<br>5.18 (s、2H、$CH_2O$)<br>6.88 (s、1H、CH=C)<br>7.10~7.48 (m、4H、aromatic H )<br>7.40 (s、1H、CH=N) |
| 13 | 1.30 (d、6H、$CH_3$-C)<br>4.20 (s、2H、$CH_2$-S)<br>5.20 (m、1H、CH-N)<br>6.76 (s、1H、CH=C)<br>7.20~7.60 (m、4H、aromatic H )<br>7.60 (s、1H、CH=N) |
| 14 | 1.30 (d、6H、$CH_3$-C)<br>5.20 (s、2H、$CH_2$-O)<br>5.22 (m、1H、CH-N)<br>6.70 (s、1H、CH=C)<br>7.16~7.60 (m、4H、aromatic H )<br>7.68 (s、1H、CH=N) |

| Compound No. | NMR ($CDCl_3$) Data , δ ppm |
|---|---|
| 15 | 1.30 (d、6H、$CH_3$ −C)<br>4.20 (s、2H、$CH_2$ −S)<br>5.20 (m、1H、CH−N)<br>6.76 (s、1H、CH=C)<br>7.20~7.60 (m、4H、aromatic H )<br>7.60 (s、1H、CH=N) |
| 16 | 1.30 (d、6H、$CH_3$ −C)<br>4.12 (s、2H、$CH_2$ S)<br>5.16 (m、1H、CH−N)<br>6.68 (s、1H、CH=C)<br>7.06~7.48 (m、4H、aromatic H )<br>7.42 (s、1H、CH=N) |
| 17 | 1.30 (d、6H、$CH_3$ −C)<br>5.20 (s、2H、$CH_2$ −O)<br>5.22 (m、1H、CH−N)<br>6.70 (s、1H、CH=C)<br>7.10~7.50 (m、4H、aromatic H )<br>7.66 (s、1H、CH=N) |
| 18 | 1.38 (d、6H、$CH_3$ −C)<br>4.20 (s、2H、$CH_2$ S)<br>3.20 (m、1H、CH−N)<br>6.70 (s、1H、CH=C)<br>7.10~7.50 (m、4H、aromatic H )<br>7.40 (s、1H、CH=N) |

| Compound No. | NMR ($CDCl_3$) Data, δ ppm |
|---|---|
| 19 | 1.36 (d、6H、$CH_3$ −C)<br>4.18 (s、2H、$CH_2$ S)<br>3.20 (m、1H、CH−N)<br>7.22 (s、1H、CH=C)<br>7.10～7.50 (m、4H、aromatic H)<br>7.52 (s、1H、CH=N) |
| 20 | 1.36 (d、6H、$CH_3$ −C)<br>4.18 (s、2H、$CH_2$ S)<br>3.20 (m、1H、CH−N)<br>6.70 (s、1H、CH=C)<br>7.14～7.50 (m、4H、aromatic H)<br>7.40 (s、1H、CH=N) |

TEST EXAMPLE 1

(two-spotted spider mites)

In 98 parts by weight of acetone was dissolved 2 parts by weight of the compound of the present invention. This solution was diluted with an aqueous solution containing 0.04% of a spreader and sticker (Trademark "Shinrino", product of Nihon Nohyaku Co., Ltd.) to a specified concentration. Kidney-bean plants in pots were inoculated with adult two-spotted spider mites (Tetranychus urticae Koch) and the above dilution was sprayed until it dripped. The mortality was estimated 3 days later. The results are shown in Table 2. The test compound No. in Table 2 corresponds to Example No.

TABLE 2

| Test Compound No. | Concentration of Test Compound (ppm) | Mortality (Adult) (%) |
|---|---|---|
| 1 | 50 | 100 |
| 3 | 50 | 100 |
| 4 | 50 | 100 |
| 5 | 50 | 100 |
| 8 | 50 | 100 |
| 10 | 50 | 100 |
| A | 50 | 90 |

A: Comparative Compound

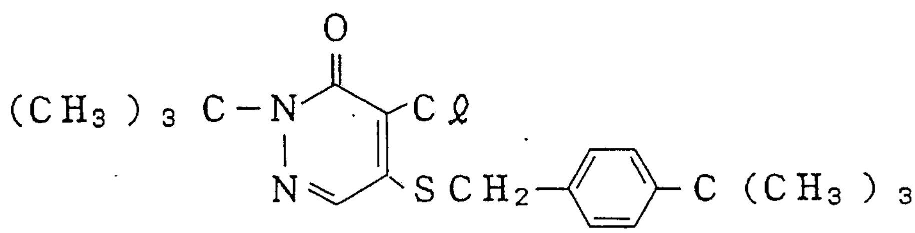

TEST EXAMPLE 2

(common cutworms)

In 98 parts by weight of acetone was dissolved 2 parts by weight of the compound of the present invention. This solution was diluted with an aqueous solution containing 0.04% of a spreader and sticker (Trademark "Shinrino", product of Nihon Nohyaku Co., Ltd.) to a specified concentration. A piece of cabbage leaf (7 cm x 7 cm) was immersed in the above dilution for 10 seconds. In a plastic cup of 13 cm in diameter were placed 4th instar larvae of common cutworms (Spodoptera litura Fabricius) together with the above cabbage piece, and the cup was left to stand in a constant temperature chamber at 25 ± 1° C. The mortality and the degree of feeding were estimated 2 days later. The results are shown in Table 3. The test compound No. in Table 3 corresponds to Example No.

TABLE 3

| Test Compound No. | Concentration of Test Compound (ppm) | Mortality (Larva) (%) | Degree of Feeding |
|---|---|---|---|
| 1 | 400 | 100 | 2 |
| 4 | 400 | 98 | 2 |
| 5 | 400 | 98 | 2 |
| 8 | 400 | 100 | 2 |
| 10 | 400 | 100 | 2 |
| 19 | 400 | 90 | 2 |
| A | 400 | 13 | 4 |
| Degree of Feeding: | | | |
| 1: No feeding<br>2: Rate of feeding of less than 10%<br>3: Rate of feeding of 10-50%<br>4: Rate of feeding of more than 50% | | | |

TEST EXAMPLE 3

(diamond back moth)

In 98 parts by weight of acetone was dissolved 2 parts by weight of the compound of the present invention. This solution was diluted with an aqueous solution containing 0.04% of a spreader and sticker (Trademark "Shinrino", product of Nihon Nohyaku Co., Ltd.) to a specified concentration. A piece of cabbage leaf (7 cm x 7 cm) was immersed in the above dilution for 10 seconds. In a plastic cup of 13 cm in diameter were placed 4th instar larvae of diamond back moths (Plutella xylostella Curtis) together with the above cabbage piece, and the cup was left to stand in a constant temperature chamber at 25 ± 1° C. The mortality after 2 days and the percent of emergence after 7 days were estimated. The results are shown in Table 4. The test compound No. in Table 4 corresponds to Example No.

TABLE 4

| Test Compound No. | Concentration of Test Compound (ppm) | Mortality (Larva) (%) | Emergence (%) |
|---|---|---|---|
| 1 | 400 | 100 | 0 |
| 4 | 400 | 97 | 0 |
| 5 | 400 | 100 | 0 |
| 8 | 400 | 100 | 0 |
| 10 | 400 | 100 | 0 |
| 18 | 400 | 97 | 0 |
| A | 400 | 47 | 53 |

TEST EXAMPLE 4

(green peach aphids)

In 98 parts by weight of acetone was dissolved 2 parts by weight of the compound of the present invention. This solution was diluted with an aqueous solution containing 0.04% of a spreader and sticker (Trademark "Shinrino", product of Nihon Nohyaku Co., Ltd.) to a specified concentration. A piece of cabbage leaf (7 cm x 7 cm) was immersed in the above dilution for 10 seconds. In a plastic Petri dish of 9 cm in diameter were placed adult green peach aphids (Myzus persicae) together with the above cabbage piece, and the dish was left to stand in a constant temperature chamber at 25 ± 1° C. The mortality was estimated 2 days later. The results are shown in Table 5. The test compound No. in Table 5 corresponds to Example No.

TABLE 5

| Test Compound No. | Concentration of Test Compound (ppm) | Mortality (Adult) (%) |
|---|---|---|
| 1 | 100 | 100 |
| 3 | 100 | 93 |
| 4 | 100 | 100 |
| 5 | 100 | 100 |
| 8 | 100 | 100 |
| 10 | 100 | 93 |
| A | 100 | 0 |

TEST EXAMPLE 5

(thrips palmi)

In 98 parts by weight of acetone was dissolved 2 parts by weight of the compound of the present invention. This solution was diluted with an aqueous solution containing 0.04% of a spreader and sticker (Trademark "Shinrino", product of Nihon Nohyaku Co., Ltd.) to a specified concentration. A piece of cucumber leaf (7 cm x 7 cm) was immersed in the above dilution for 10 seconds. In a plastic cup of 9 cm in diameter were placed male imagines and larvae of thrips palmi in 2nd stage together with the above cucumber leaf piece, and the cup was left to stand in a constant temperature chamber at 25 ± 1° C. The mortality after 2 days and the percent of emergence after 7 days were estimated. The results are shown in Table 6. The test compound No. in Table 6 corresponds to Example No.

TABLE 6

| Test Compound No. | Concentration of Test Compound (ppm) | Mortality (%) | | Emergence (%) |
|---|---|---|---|---|
| | | Adult | Larva | |
| 1 | 100 | 100 | 60 | 8 |
| 5 | 100 | 97 | 50 | 17 |
| 8 | 100 | 100 | 55 | 15 |
| 10 | 100 | 97 | 60 | 10 |
| 15 | 100 | 90 | 50 | 18 |
| 18 | 100 | 90 | 50 | 16 |
| A | 100 | 50 | 63 | 53 |

TEST EXAMPLE 6

(cucmber powdery mildew)

In 98 parts by weight of acetone was dissolved 2 parts by weight of the compound of the present invention. This solution was diluted with an aqueous solution containing 0.01% of a spreader and sticker (Tween 80) to a specified concentration. The dilution was sprayed onto cucumber seedlings in two-leaf stage planted in pots until the dilution dripped. After air-drying, spores of Sphaerotheca fuliginea causing the above disease were innoculated. Ten days later, the infection degree was determined from the infection index according to the criteria given below. The preventive value was calculated from the following equation.
Infection index A: All over the leaf surface is infected.
B: More than 2/3 part of the leaf surface is infected.
C: 1/3 to 2/3 part of the leaf surface is infected.
D: Less than 1/3 part of the leaf surface is infected.
E: No infection is observed.

$$\text{Infection degree} = \frac{4A + 3B + 2C + D}{4 \times (\text{number of leaves tested})} \times 100$$

$$\text{Percent inhibitory effect} = \frac{X - Y}{X} \times 100$$

where X is the infection degree of untreated group and Y is the infection degree of treated group.
The results are shown in Table 7.

TABLE 7

| Test Compound No. | Concentration of Test Compound (ppm) | Preventive Value (%) | Phytotoxicity |
|---|---|---|---|
| 1 | 100 | 95 | None |
| 5 | 100 | 98 | None |
| 8 | 100 | 92 | None |
| 9 | 100 | 92 | None |
| 10 | 100 | 96 | None |
| 20 | 100 | 90 | None |
| A | 100 | 96 | None |
| B | 400 | 95 | None |
| B: Comparative Compound (Tradename "Molestan", product of Nihon Tokushu Nohyaku Co., Ltd.) | | | |

**Claims**

1. A pyridazinone derivative represented by the formula

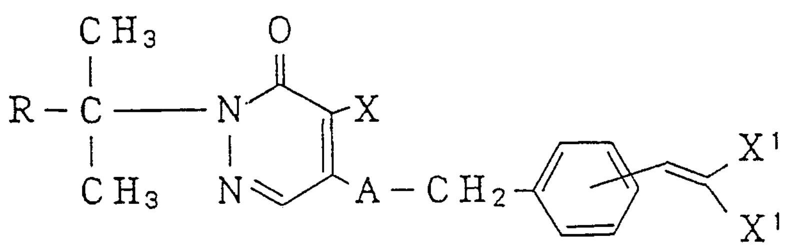

(I)

wherein R is a hydrogen atom or methyl, X is a halogen atom, A is an oxygen atom or a sulfur atom, and $X^1$ is a halogen atom.

2. A pyridazinone derivative according to Claim 1 which is selected from
2-t-Butyl-4-chloro-5-[4-(β,β-dichlorovinyl)]benzylthio-3(2H)-pyridazinone,
2-t-Butyl-4-chloro-5-[4-(β,β-dichlorovinyl)]benzyloxy-3(2H)-pyridazinone,
2-t-Butyl-4-chloro-5-[4-(β,β-dibromovinyl)]benzylthio-3(2H)-pyridazinone,
2-t-Butyl-4-chloro-5-[3-(β,β-dichlorovinyl)]benzylthio-3(2H)-pyridazinone,
2-t-Butyl-4-chloro-5-[2-(β,β-dichlorovinyl)]benzylthio-3(2H)-pyridazinone,
2-t-Butyl-4-chloro-5-[3-(β,β-dichlorovinyl)] benzyloxy-3(2H)-pyridazinone,
2-t-Butyl-4-chloro-5-[2-(β,β-dichlorovinyl)]benzyloxy-3(2H)-pyridazinone,
2-t-Butyl-4-bromo-5-[4-(β,β-dichlorovinyl)]benzylthio-3(2H)-pyridazinone,
2-t-Butyl-4-bromo-5-[4-(β,β-dibromovinyl)]benzylthio-3(2H)-pyridazinone,
2-t-Butyl-4-bromo-5-[2-(β,β-dichlorovinyl)]benzylthio-3(2H)-pyridazinone,
2-t-Butyl-4-bromo-5-[4-(β,β-dichlorovinyl)]benzyloxy-3(2H)-pyridazinone,
2-t-Butyl-4-bromo-5-[2-(β,β-dichlorovinyl)]benzyloxy-3(2H)-pyridazinone,
2-Isopropyl-4-chloro-5-[4-(β,β-dichlorovinyl)]benzylthio-3(2H)-pyridazinone,
2-Isopropyl-4-chloro-5-[4-(β,β-dichlorovinyl)]benzyloxy-3(2H)-pyridazinone,
2-Isopropyl-4-chloro-5-[2-(β,β-dichlorovinyl)]benzylthio-3(2H)-pyridazinone,
2-Isopropyl-4-chloro-5-[3-(β,β-dichlorovinyl)]benzylthio-3(2H)-pyridazinone,
2-Isopropyl-4-chloro-5-[3-(β,β-dichlorovinyl)]benzyloxy-3(2H)-pyridazinone,
2-Isopropyl-4-bromo-5-[2-(β,β-dichlorovinyl)]benzylthio-3(2H)-pyridazinone,
2-Isopropyl-4-chloro-5-[4-(β,β-dibromovinyl)]benzylthio-3(2H)-pyridazinone and
2-Isopropyl-4-bromo-5-[4-(β,β-dichlorovinyl)]benzylthio-3(2H)-pyridazinone.

3. A insectidical, miticidal and fungicidal composition for agricultural and horticultural use comprising an

effective amount of at least one pyridazinone derivative according to claims 1 or 2.

4. A method for killing harmful insects, mites and bacteria comprising applying to a plant an effective amount of at least one pyridazinone derivative according to claims 1 or 2.